# EUROPEAN PATENT APPLICATION

(11) **EP 4 653 513 A2**
(43) Date of publication of application: **26.11.2025**
(21) Application number: 25207538.7
(22) Date of filing: 03.12.2020
(51) Int. Cl.: C09K 23/32

(54) **METAL DOUBLE SALT LIQUID DISPERSION, METHOD FOR PRODUCING METAL DOUBLE SALT LIQUID DISPERSION**

(30) Priority: 28.02.2020 JP 2020033217
(62) Divisional of application: 20922345.2
(71) Applicant: Murata Manufacturing Co., Ltd., Nagaokakyo-shi, Kyoto 617-8555 (JP)
(72) Inventor: OTSUKA, Yusuke, Nagaokakyo-shi, Kyoto, 617-8555 (JP)
(74) Representative: Lorenz Seidler Gossel Part. mbB

(57) **Abstract**

The present invention relates to a metal oxide nanoparticle dispersion liquid comprising: metal oxide nanoparticles; and an organic base having an amidine skeleton or a guanidine skeleton, wherein a molar ratio of a content of the organic base to a content of a metal component constituting the metal oxide nanoparticles is 0.03 or less.

## Description

### TECHNICAL FIELD

The present invention relates to a metal double salt dispersion liquid, a method for producing a metal double salt dispersion liquid, a metal oxide nanoparticle dispersion liquid, and a method for producing a metal oxide nanoparticle dispersion liquid.

### BACKGROUND ART

Metal oxide fine particles are used in various fields taking advantage of characteristics such as low temperature sinterability, high specific surface area, dispersibility in a solvent, and quantum effect.

Examples of a method for producing metal oxide fine particles include a build-down method in which a bulk metal oxide is pulverized and a build-up method in which particles are grown at a molecular level. There is a limit to miniaturization by the build-down method, and usually the build-up method is used at present.

As a method for producing metal oxide particles, Patent Document 1 discloses a method including simultaneously adding an aqueous solution of a metal salt and an aqueous solution of a neutralizing agent to an aqueous solution of a carboxylic acid compound to form fine particles of a metal hydroxide or hydrate, and firing the fine particles.

Non-Patent Document 1 discloses a method using lithium hydroxide as a base as a method for producing a dispersion liquid in which zinc oxide nanoparticles are dispersed in ethanol.

### PRIOR ART DOCUMENTS

### PATENT DOCUMENT

Patent Document 1: Japanese Patent Application Laid-Open No. 2005-139704

### NON-PATENT DOCUMENT

Non-Patent Document 1: E. A. Meulenkamp, J. Phys. Chem. B, 102, 5566, (1998)

### SUMMARY OF THE INVENTION

### Problem to be solved by the invention

The method described in Patent Document 1, however, has a problem that the resulting oxide particles have a large particle size and poor dispersibility in a solvent because it is necessary to fire the metal hydroxide or the metal hydrate.

Because the method described in Patent Document 1 needs to fire a metal hydroxide or a metal hydrate, a method for obtaining oxide fine particles without firing has been required.

The zinc oxide nanoparticles produced by the method described in Non-Patent Document 1 have high dispersibility immediately after production but have a problem that the dispersibility decreases with the lapse of time.

The inventors of the present invention have extensively conducted studies and have found that the above problems can be solved by using a dispersion liquid of a double salt obtained by reacting a metal carboxylate with a base.

That is, an object of the present invention is to provide a metal double salt dispersion liquid with which a dispersion liquid of metal oxide nanoparticles having a small particle size and high dispersibility can be produced without requiring firing, and a method for producing the metal double salt dispersion liquid, and to provide a metal oxide nanoparticle dispersion liquid excellent in temporal stability of dispersibility, and a method for producing the metal oxide nanoparticle dispersion liquid.

### Means for solving the problem

A metal double salt dispersion liquid of the present invention is a metal double salt dispersion liquid including an organic solvent and a metal double salt, wherein the metal double salt has a composition represented by M(R¹COO)_{m-x-y}(OH)ₓA_{y}(H₂O)z, where M is a metal element, R¹ is a hydrogen atom or an alkyl group, A is an anion, m is a valence of the metal element M, and 0 < x + y <m, x > 0, y ≥ 0, and z ≥ 0 are satisfied, and when the metal double salt dispersion liquid is subjected to a centrifugal operation at a relative centrifugal force of 10,000 G for 5 minutes, a proportion of metal elements not forming a precipitate to all metal elements contained in a total of the metal double salt dispersion liquid is 10.0 mol% or more.

A method for producing a metal double salt dispersion liquid of the present invention includes a step of adding a strong base to a metal salt dispersion liquid including a metal carboxylate and an organic solvent, wherein when a metal element constituting the metal carboxylate has a valence m, the strong base has a substance amount with respect to a substance amount of the metal carboxylate of 0.4m or more and 0.9m or less.

A metal oxide nanoparticle dispersion liquid of the present invention includes metal oxide nanoparticles and an organic base having an amidine skeleton or a guanidine skeleton.

A method for producing a metal oxide nanoparticle dispersion liquid of the present invention includes a preparation step of preparing a metal double salt dispersion liquid by adding a strong base to a metal salt dispersion liquid including a metal carboxylate and an organic solvent, and a heating step of heating the metal double salt dispersion liquid in a presence of water to obtain a metal oxide nanoparticle dispersion liquid, wherein in the preparation step, when a metal element constituting the metal carboxylate has a valence m, the strong base has a substance amount with respect to a substance amount of the metal carboxylate of 0.4m or more and 0.9m or less, and the strong base includes an organic base having an amidine skeleton or a guanidine skeleton.

### Advantageous effect of the invention

The present invention can provide a metal double salt dispersion liquid with which a dispersion liquid of metal oxide nanoparticle dispersion liquid having a small particle size and high dispersibility can be produced without requiring firing, and a method for producing the metal double salt dispersion liquid.

The present invention can also provide a metal oxide nanoparticle dispersion liquid excellent in temporal stability of dispersibility of metal oxide nanoparticles, and a method for producing the metal oxide nanoparticle dispersion liquid.

### BRIEF EXPLANATION OF DRAWINGS

FIG. 1 is a photograph of metal double salt dispersion liquids according to Example 1 and Comparative Example 1 after being subjected to a centrifugal operation.
FIG. 2 is a photograph of metal oxide nanoparticle dispersion liquids according to Example 1 and Comparative Example 1 subjected to a centrifugal operation.
FIG. 3 is a TEM image of a metal double salt according to Example 15.
FIG. 4 is a TEM image of the metal double salt according to Example 15.
FIG. 5 is a spectrum showing a particle size distribution of particles contained in a metal double salt dispersion liquid according to Example 15.

### MODE FOR CARRYING OUT THE INVENTION

Hereinafter, the metal double salt dispersion liquid and the metal oxide nanoparticle dispersion liquid of the present invention will be described.

The present invention is not limited to the following configurations and can be appropriately modified and applied without changing the spirit of the present invention. The present invention also includes a combination of two or more of individual desirable configurations described below.

### [Metal double salt dispersion liquid]

A metal double salt dispersion liquid of the present invention is a metal double salt dispersion liquid including an organic solvent and a metal double salt, wherein the metal double salt has a composition represented by M(R¹COO)_{m-x-y}(OH)ₓA_{y}(H₂O)z, where M is a metal element, R¹ is a hydrogen atom or an alkyl group, A is an anion, m is a valence of the metal element M, and 0 < x + y <m, x > 0, y ≥ 0, and z ≥ 0 are satisfied, and when the metal double salt dispersion liquid is subjected to a centrifugal operation at a relative centrifugal force of 10,000 G for 5 minutes, a proportion of metal elements not forming a precipitate to all metal elements contained in a total of the metal double salt dispersion liquid is 10.0 mol% or more.

The metal double salt dispersion liquid of the present invention includes a metal double salt having a composition represented by M(R¹COO)_{m-x-y}(OH)ₓA_{y}(H₂O)z, where M is a metal element, R¹ is a hydrogen atom or an alkyl group, A is an anion, m is a valence of the metal element M, and 0 < x + y <m, x > 0, y ≥ 0, and z ≥ 0 are satisfied.

Including carboxylate (R¹COO⁻), the metal double salt is excellent in affinity for an organic solvent and has high dispersibility in an organic solvent. Including hydroxide ion (OH⁻), the metal double salt can be easily converted into a metal oxide by heating at less than 100°C.

When the metal double salt dispersion liquid of the present invention is subjected to a centrifugal operation at a relative centrifugal force of 10,000 G for 5 minutes, a proportion of metal elements not forming a precipitate to all metal elements contained in a total of the metal double salt dispersion liquid is 10.0 mol% or more. That is, in the metal double salt dispersion liquid of the present invention, 10.0 mol% or more of all metal elements are dispersed in the solution when a predetermined centrifugal operation is performed, and it can be said that dispersibility of the metal double salt is high.

When the metal double salt dispersion liquid of the present invention is subjected to a centrifugal operation at a relative centrifugal force of 10,000 G for 5 minutes, the proportion of metal elements not forming a precipitate to all metal elements contained in the total of the metal double salt dispersion liquid is preferably 12.6 mol% or more, and more preferably 30.0 mol% or more.

In the metal double salt dispersion liquid of the present invention, the metal element M constituting the metal double salt preferably includes at least one metal selected from the group consisting of Cu, Mn, Co, Ce, Fe, and In.

When the metal element M includes two or more metal elements having different valences, the sum of values obtained by multiplying the abundance proportion [mol%] of each metal element in the total metal elements by the valence of each metal element is defined as the valence m of the metal element.

The functional group R¹ constituting the metal double salt preferably includes at least one functional group selected from the group consisting of a hydrogen atom, a methyl group, an ethyl group, a 1-propyl group, and a 2-propyl group.

Among them, a methyl group is preferable.

The metal double salt dispersion liquid of the present invention may be a mixture of two or more types of double salts having different R¹.

Examples of the anion A constituting the metal double salt include a chloride ion (Cl⁻), a nitrate ion (NO₃⁻), a carbonate ion (CO₃²⁻), and a sulfate ion (SO₄²⁻)

When the proportion of the anion A is large, the proportion of the carboxylate relatively decreases, and thus the dispersibility of the double salt deteriorates. Thus, y is preferably 0 or more and 1 or less.

In the composition formula representing the metal double salt, z represents the number of bonds of hydration water.

When the number of bonds of hydration water is large, the dispersibility of the double salt deteriorates. Thus, z is preferably 0 or more and 4 or less.

The metal double salt dispersion liquid of the present invention may include a salt of a carboxylic acid and a strong base or a metal carboxylate in addition to the organic solvent and the metal double salt.

The salt of a carboxylic acid and a strong base is a by-product produced in the process of producing the metal double salt dispersion liquid of the present invention.

The metal carboxylate is an unreacted raw material used in the process of producing the metal double salt dispersion liquid of the present invention.

Examples of the salt of a carboxylic acid and a strong base include tetramethylammonium acetate, diazabicycloundecene acetate, diazabicyclononene acetate, tetramethylguanidine acetate, and tetraethylammonium acetate.

Among them, diazabicycloundecene acetate is preferable.

Examples of the strong base include alkali metal hydroxides such as lithium hydroxide, sodium hydroxide, potassium hydroxide, and cesium hydroxide, quaternary ammonium hydroxides such as tetramethylammonium hydroxide (TMAH) and tetraethylammonium hydroxide (TEAH), organic bases having an amidine skeleton such as 1,8-diazabicyclo[5.4.0]undec-7-ene (also referred to as diazabicycloundecene or DBU), 1,5-diazabicyclo[4.3.0]non-5-ene (also referred to as diazabicyclonene or DBN), 1,5,7-triazabicyclo[4.4.0]dec-5-ene, and 7-methyl-1,5,7-triazabicyclo[4.4.0]dec-5-ene, and organic bases having a guanidine skeleton such as 1,1,3,3-tetramethylguanidine (also referred to as tetramethylguanidine or TMG).

Among them, an organic base having an amidine skeleton or a guanidine skeleton is preferable, and diazabicycloundecene is more preferable.

In the present specification, a strong base refers to a base having a base dissociation constant (pKb) of 2 or less in water, or a base whose conjugate acid has an acid dissociation constant (pKa) of 12 or more in water.

The carboxylic acid preferably has a composition represented by R²COOH, where R² is a hydrogen atom or an alkyl group.

The functional group R² constituting the carboxylic acid is preferably the same as the functional group R¹ constituting the metal double salt.

Examples of the metal carboxylate include copper (II) acetate, manganese (II) acetate, cobalt (II) acetate, cerium (III) acetate, iron (II) acetate, and indium (III) acetate.

The metal element concentration in the metal double salt dispersion liquid of the present invention is not particularly limited but is preferably 0.0001 mol/L or more and 2.0 mol/L or less.

The type of the organic solvent used in the metal double salt dispersion liquid of the present invention is not particularly limited, but it is desirable that the organic solvent has a Snydel polarity parameter of 3.5 or more.

Examples of the organic solvent having a Snydel polarity parameter of 3.5 or more include alcohols such as methanol and ethanol, dimethyl sulfoxide (DMSO), dimethylformamide (DMF), dimethylacetamide (DMAc), 1-methyl-2-pyrrolidone (NMP), 1,3-dimethyl-2-imidazolidinone (DMI), and pyridine.

The average particle size of the metal double salt contained in the metal double salt dispersion liquid of the present invention in a dry state is preferably 1 nm or more and 5 nm or less.

The average particle size is an average value of particle sizes of 30 metal double salt particles randomly selected from a visual field, the particle sizes being able to be measured with a transmission electron microscope (TEM).

The metal double salt dispersion liquid of the present invention can be formed into a metal oxide nanoparticle dispersion liquid by heating in the presence of water.

In the metal oxide nanoparticle dispersion liquid produced using the metal double salt dispersion liquid of the present invention, carboxylate derived from the double salt is present on the surfaces of the metal oxide nanoparticles, and therefore it is considered that the metal oxide nanoparticles are colloidally dispersed in an organic solvent and are excellent in dispersibility.

When the metal double salt dispersion liquid of the present invention is heated, 2 mol or more and 20 mol or less of water is preferably present per 1 mol of the metal double salt.

When the abundance of water is more than 20 mol with respect to 1 mol of the metal double salt, dispersibility of the metal oxide obtained by heating the metal double salt may deteriorate.

Examples of a method for producing the metal oxide nanoparticle dispersion liquid using the metal double salt dispersion liquid of the present invention includes a method of heating the metal double salt dispersion liquid at a temperature of 50°C or more and less than 100°C for 15 minutes or more and 12 hours or less in a state where water is added to the metal double salt dispersion liquid.

The metal double salt dispersion liquid of the present invention can also be used for applications other than the above-described applications for producing a metal oxide nanoparticle dispersion liquid. For example, a thin film of a metal oxide can be formed by forming a film containing a metal double salt using the metal double salt dispersion liquid of the present invention and heating the film. A material in which a base material and a metal double salt are combined can also be obtained by mixing the metal double salt dispersion liquid of the present invention with a substance serving as the base material.

### [Method for producing metal double salt dispersion liquid]

A method for producing a metal double salt dispersion liquid of the present invention includes a step of adding a strong base to a metal salt dispersion liquid including a metal carboxylate and an organic solvent, wherein when a metal element constituting the metal carboxylate has a valence m, the strong base has a substance amount with respect to a substance amount of the metal carboxylate of 0.4m or more and 0.9m or less.

In the method for producing a metal double salt dispersion liquid of the present invention, a strong base is added to a metal salt dispersion liquid including a metal carboxylate and an organic solvent such that the strong base has a substance amount with respect to a substance amount of the metal carboxylate of 0.4m or more and 0.9m or less when the valence of the metal element constituting the metal carboxylate is m. The metal carboxylate dispersed in the organic solvent therefore reacts with the strong base to form a metal double salt.

Including carboxylate (R¹COO⁻), the metal double salt obtained by reacting a metal carboxylate with a strong base is excellent in affinity for an organic solvent and has high dispersibility in an organic solvent. Including hydroxide ion (OH⁻), the double salt can be easily converted into a metal oxide by heating at less than 100°C.

A metal double salt dispersion liquid having high dispersibility and reactivity can be therefore produced by the method for producing a metal double salt dispersion liquid of the present invention.

When the proportion of the strong base is less than 0.4m, the formed metal double salt is hardly converted into an oxide. On the other hand, when the proportion of the strong base exceeds 0.9m, the dispersibility of the formed metal double salt is not sufficient.

Examples of the metal carboxylate used in the method for producing a metal double salt dispersion liquid of the present invention include copper (II) acetate, manganese (II) acetate, cobalt (II) acetate, cerium (III) acetate, iron (II) acetate, and indium (III) acetate. These acetates may be hydrates. In addition, a plurality of types of metal elements may be included.

When the metal carboxylate contains two or more metal elements having different valences, the sum of values obtained by multiplying the abundance proportion [mol%] of each metal element in the total metal elements by the valence of each metal element is defined as the valence m of the metal element constituting the metal carboxylate.

As the organic solvent and the strong base used in the method for producing a metal double salt dispersion liquid of the present invention, those like the organic solvent and the strong base constituting the metal double salt dispersion liquid of the present invention may be used.

The metal salt dispersion liquid may be obtained by mixing a metal carboxylate with an organic solvent.

As a method for preparing the metal salt dispersion liquid, a method using no metal carboxylate may also be used.

When the metal salt dispersion liquid is prepared without using a metal carboxylate, for example, a metal salt (for example, chloride, nitrate, sulfate, carbonate, etc.) other than a metal carboxylate, a carboxylic acid compound, and an organic solvent may be mixed.

In the step of adding a strong base to the metal salt dispersion liquid, it is preferable to add a strong base dropwise while mixing the metal salt dispersion liquid.

The strong base may be added to the metal salt dispersion liquid in a state of being dissolved or dispersed in an organic solvent.

One type of the strong base may be added, or two or more types of the strong base may be added.

### [Metal oxide nanoparticle dispersion liquid]

A metal oxide nanoparticle dispersion liquid of the present invention includes metal oxide nanoparticles and an organic base having an amidine skeleton or a guanidine skeleton.

The metal oxide nanoparticle dispersion liquid of the present invention contains an organic base having an amidine skeleton or a guanidine skeleton. The temporal stability of the dispersibility of the metal oxide nanoparticles in the metal oxide nanoparticle dispersion liquid can be thus improved.

Examples of the organic base having an amidine skeleton include 1,8-diazabicyclo[5.4.0]undec-7-ene (also referred to as diazabicycloundecene or DBU), 1,5-diazabicyclo[4.3.0]non-5-ene (also referred to as diazabicyclononene or DBN), 1,5,7-triazabicyclo[4.4.0]dec-5-ene, and 7-methyl-1,5,7-triazabicyclo[4.4.0]dec-5-ene. Among them, diazabicycloundecene and diazabicyclononene are preferable.

Examples of the organic base having a guanidine skeleton include 1,1,3,3-tetramethylguanidine (also referred to as tetramethylguanidine or TMG).

Thus, the organic base preferably includes at least one compound selected from the group consisting of diazabicycloundecene, diazabicyclononene, and tetramethylguanidine.

When the organic base includes at least one compound selected from the group consisting of diazabicycloundecene, diazabicyclononene, and tetramethylguanidine, it is presumed that the metal oxide nanoparticles to which these compounds are attached are prevented from approaching each other and are less likely to aggregate, and the temporal stability of the dispersibility of the metal oxide nanoparticles increases, because these compounds have a large steric hindrance.

The molar ratio of the content of the organic base to the content of a metal component constituting the metal oxide nanoparticles is preferably 0.03 or less.

When the molar ratio of the content of the organic base exceeds 0.03, the proportion of impurities contained in the metal oxide nanoparticle dispersion liquid is small, and it is possible to inhibit occurrence of inconvenience because of impurities when the metal oxide nanoparticle dispersion liquid is used.

The content of the organic base having an amidine skeleton or a guanidine skeleton contained in the metal oxide nanoparticle dispersion liquid may be measured by gas chromatography.

The molar ratio of the content of the organic base to the content of the metal component constituting the metal oxide nanoparticles may be 0.001 or more and is equal to or more than the detection limit of the organic base.

The organic base preferably includes at least one compound selected from the group consisting of diazabicycloundecene, diazabicyclononene, and tetramethylguanidine.

The metal component constituting the metal oxide nanoparticles preferably includes at least one metal selected from the group consisting of Cu, Mn, Co, Ce, Fe, and In. The metal oxide nanoparticles may include two or more types of metals.

The type of the metal component constituting the metal oxide nanoparticles and the type of the metal oxide are not related to the dispersibility of the metal oxide nanoparticles in the metal oxide nanoparticle dispersion liquid.

The average particle size of the metal oxide nanoparticles constituting the metal oxide nanoparticle dispersion liquid is preferably 1 nm or more and 20 nm or less, and more preferably 1 nm or more and 10 nm or less.

When the average particle size of the metal oxide nanoparticles is within the above range, the metal oxide nanoparticles are excellent in temporal stability of dispersibility.

The average particle size of the metal oxide nanoparticles is a particle size (D50) of the metal oxide nanoparticles at a cumulative number of 50% as measured by a dynamic light scattering method.

### [Method for producing metal oxide nanoparticle dispersion liquid]

A method for producing a metal oxide nanoparticle dispersion liquid of the present invention includes a preparation step of preparing a metal double salt dispersion liquid by adding a strong base to a metal salt dispersion liquid including a metal carboxylate and an organic solvent, and a heating step of heating the metal double salt dispersion liquid in a presence of water to obtain a metal oxide nanoparticle dispersion liquid, wherein in the preparation step, when a metal element constituting the metal carboxylate has a valence m, the strong base has a substance amount with respect to a substance amount of the metal carboxylate of 0.4m or more and 0.9m or less, and the strong base includes an organic base having an amidine skeleton or a guanidine skeleton.

Using the method for producing a metal oxide nanoparticle dispersion liquid of the present invention enables a metal oxide nanoparticle dispersion liquid excellent in temporal stability of dispersibility to be produced.

The metal oxide nanoparticle dispersion liquid produced by the method for producing a metal oxide nanoparticle dispersion liquid of the present invention is also the metal oxide nanoparticle dispersion liquid of the present invention.

The method for producing a metal oxide nanoparticle dispersion liquid of the present invention corresponds to a method for heating, in the method for producing a metal double salt dispersion liquid of the present invention, the resulting metal double salt dispersion liquid in the presence of water using an organic base having an amidine skeleton or a guanidine skeleton as a strong base.

Thus, the description of the step of preparing the metal double salt dispersion liquid is omitted.

The organic base preferably includes at least one compound selected from the group consisting of diazabicycloundecene, diazabicyclononene, and tetramethylguanidine.

Diazabicycloundecene, diazabicyclononene, and tetramethylguanidine can improve the temporal stability of the dispersion of the metal oxide nanoparticles in the metal oxide nanoparticle dispersion liquid. In addition, it is easy to specify that diazabicycloundecene, diazabicyclononene, and tetramethylguanidine are contained in the metal oxide nanoparticle dispersion liquid.

The metal component constituting the metal oxide nanoparticles preferably includes at least one metal selected from the group consisting of Cu, Mn, Co, Ce, Fe, and In.

The water may be water added in the process of preparing the metal double salt dispersion liquid, or water separately added after the preparation of the metal double salt dispersion liquid. Examples of the water added in the process of preparing the metal double salt dispersion liquid include hydration water included in the metal carboxylate.

The abundance of water when the metal double salt dispersion liquid is heated is preferably 2 times or more and 20 times or less in terms of molar ratio with respect to the metal component.

The temperature and time for heating the metal double salt dispersion liquid in the coexistence of water may be appropriately adjusted depending on the type and particle size of the metal oxide nanoparticles to be obtained.

The heating temperature is, for example, 50°C or more and less than 100°C.

The heating time is, for example, 15 minutes or more and 12 hours or less.

The metal oxide nanoparticle dispersion liquid obtained by the above procedure may be purified as necessary.

Purifying the metal oxide nanoparticle dispersion liquid can reduce the content of the organic base contained in the metal oxide nanoparticle dispersion liquid.

Examples of the procedure for purifying the metal oxide nanoparticle dispersion liquid include a method in which an organic solvent such as methyl acetate is added to the metal oxide nanoparticle dispersion liquid, then centrifugation treatment is performed to precipitate the metal oxide nanoparticles, a supernatant solution is removed, and thereafter the metal oxide nanoparticles are dispersed again in the dispersion medium. The above purification may be performed multiple times. Increasing the number of times of purification can reduce the molar ratio of the content of the organic base to the content of the metal component constituting the metal oxide nanoparticles.

The purification of the metal oxide nanoparticle dispersion liquid by the above procedure is preferably repeated until the molar ratio of the content of the organic base to the content of the metal component constituting the metal oxide nanoparticles becomes 0.03 or less.

### EXAMPLES

Hereinafter, Examples that more specifically disclose the metal double salt dispersion liquid of the present invention, the method for producing a metal double salt dispersion liquid of the present invention, the metal oxide nanoparticle dispersion liquid of the present invention, and the method for producing a metal oxide nanoparticle dispersion liquid of the present invention will be described. The present invention is not limited only to these Examples.

### <Example 1>

### (Production of metal double salt dispersion liquid)

Ethanol [super dehydration grade, manufactured by FUJIFILM Wako Pure Chemical Corporation] in an amount of 9.23 mL was added to 0.2 g of copper (II) acetate monohydrate [manufactured by FUJIFILM Wako Pure Chemical Corporation], and they were mixed at room temperature. To this solution, 0.422 mL of a 25% methanol solution of tetramethylammonium hydroxide (TMAH) [manufactured by Sigma-Aldrich Co. LLC] was added dropwise and mixed at room temperature to obtain a metal double salt dispersion liquid according to Example 1. The substance amount of strong base with respect to the substance amount of metal carboxylate was 1.00 and was 0.4m or more and 0.9m or less (m = 2).

It is considered that the metal double salt dispersion liquid according to Example 1 contains a copper double salt [Cu(CH₃COO)₂₋ₓ(OH)ₓ] as a metal double salt, copper (II) acetate as an unreacted metal carboxylate, and tetramethylammonium acetate as a salt of a carboxylic acid and a strong base.

The metal double salt powder separated from the obtained metal double salt dispersion liquid was measured by Fourier transform infrared spectroscopy (FT-IR), and from the obtained spectrum, absorption at around 1400 cm⁻¹ and around 1600 cm⁻¹ derived from carboxylate (R¹COO⁻) and absorption at around 3200 to 3500 cm⁻¹ derived from hydroxide ion (OH⁻) were confirmed.

(Measurement of amount of metal in dispersed state in metal double salt dispersion liquid)

The metal double salt dispersion liquid according to Example 1 was subjected to a centrifugal operation at a relative centrifugal force of 10,000 G for 5 minutes, and the supernatant was separated. Methyl acetate having the same volume as the separated liquid and heptane in an amount of 4 times the volume of the separated liquid were added to the separated liquid and stirred. Then, the mixture was further subjected to a centrifugal operation at a relative centrifugal force of 10,000 G for 5 minutes, thereafter the supernatant was discarded, and the residue was recovered. The proportion of the metal element (that is, double salt in a dispersed state) in which no precipitate was formed in the metal double salt dispersion liquid was calculated from the weight of copper oxide (CuO) obtained by thermally decomposing the recovered residue at 400°C. The results are shown in Table 1.

### (Production of metal oxide nanoparticle dispersion liquid)

Pure water in an amount of 0.131 mL of was added to 5 mL of the metal double salt dispersion liquid according to Example 1, and the mixture was heated and mixed in an oil bath at 70°C for 30 minutes to obtain a metal oxide nanoparticle dispersion liquid. The obtained metal oxide nanoparticle dispersion liquid was separated and then dried, and the solid content was measured by powder X-ray diffraction (XRD), and it was confirmed that there was a peak derived from copper oxide (CuO).

### (Measurement of amount of metal in dispersion state in metal oxide nanoparticle dispersion liquid)

Subsequently, methyl acetate having the same volume as the obtained metal oxide nanoparticle dispersion liquid and heptane in an amount of 4 times the volume of the obtained metal oxide nanoparticle dispersion liquid were added to the obtained metal oxide nanoparticle dispersion liquid and stirred. Then, the mixture was further subjected to a centrifugal operation at a relative centrifugal force of 10,000 G for 5 minutes, thereafter the supernatant was discarded, and the residue was recovered. The proportion of the metal element (that is, oxide in a dispersed state) that did not form a precipitate in the metal oxide nanoparticle dispersion liquid was calculated from the weight of copper oxide (CuO) obtained by thermally decomposing the recovered residue at 400°C. The results are shown in Table 1.

### <Example 2>

A metal double salt dispersion liquid according to Example 2 was produced through the same procedure as in Example 1 except that the addition amount of ethanol was changed from 9.23 mL to 8.92 mL, and the addition amount of a 25% methanol solution of TMAH was changed from 0.422 mL to 0.739 mL, then the proportion of the double salt in a dispersed state was measured. In addition, a metal oxide nanoparticle dispersion liquid was produced through the same procedure as in Example 1, and the proportion of the oxide in a dispersed state was measured. The results are shown in Table 1. The substance amount of strong base with respect to the substance amount of metal carboxylate was 1.75.

### <Comparative Example 1>

A metal double salt dispersion liquid according to Comparative Example 1 was produced through the same procedure as in Example 1 except that the addition amount of ethanol was changed from 9.23 mL to 8.90 mL, and the addition amount of a 25% methanol solution of TMAH was changed from 0.422 mL to 0.802 mL, and the proportion of the double salt in a dispersed state was measured. In addition, a metal oxide nanoparticle dispersion liquid was produced through the same procedure as in Example 1, and the proportion of the oxide in a dispersed state was measured. The results are shown in Table 1.

### <Example 3>

### (Production of metal double salt dispersion liquid)

DMSO [super dehydration grade, manufactured by FUJIFILM Wako Pure Chemical Corporation] in an amount of 7.27 mL was added to 0.2 g of copper (II) acetate tetrahydrate [manufactured by FUJIFILM Wako Pure Chemical Corporation], and they were mixed at room temperature. To this solution, 0.406 mL of a 25% methanol solution of TMAH [manufactured by Sigma-Aldrich Co. LLC] was added dropwise and mixed at room temperature to obtain a metal double salt dispersion liquid according to Example 3.

The obtained metal double salt dispersion liquid was subjected to a centrifugal operation at a relative centrifugal force of 10,000 G for 5 minutes, and the supernatant was separated. Methyl acetate in an amount of 4 times the volume of the separated liquid and toluene in an amount of 4 times the volume of the separated liquid were added to the separated liquid and stirred. Then, the mixture was subjected to a centrifugal operation at a relative centrifugal force of 10,000 G for 5 minutes, thereafter the supernatant liquid was discarded, and the residue was recovered. The proportion of the double salt in a dispersed state in the metal double salt dispersion liquid was measured from the weight of cobalt oxide (Co₃O₄) obtained by thermally decomposing the recovered residue at 400°C. The results are shown in Table 1.

### (Production of metal oxide nanoparticle dispersion liquid)

Pure water in an amount of 0.148 mL was added to 5 mL of the metal double salt dispersion liquid according to Example 3, and the mixture was heated in an oil bath at 95°C for 2.5 hours to obtain a metal oxide nanoparticle dispersion liquid according to Example 3. The obtained metal oxide nanoparticle dispersion liquid was subjected to a centrifugal operation at a relative centrifugal force of 10,000 G for 5 minutes, thereafter the supernatant was separated, and methyl acetate in an amount of 4 times the volume of the separated liquid was added to the separated liquid and stirred. Then, the mixture was further subjected to a centrifugal operation at a relative centrifugal force of 10,000 G for 5 minutes, thereafter the supernatant was discarded, and the residue was collected. The proportion of the oxide in a dispersed state in the metal oxide nanoparticle dispersion liquid was calculated from the weight of cobalt oxide (Co₃O₄) obtained by thermally decomposing the recovered residue at 400°C. The results are shown in Table 1.

### <Example 4>

### (Production of metal double salt dispersion liquid)

A metal double salt dispersion liquid according to Example 4 was produced through the same procedure as in Example 3 except that 0.2 g of manganese (II) acetate tetrahydrate [manufactured by FUJIFILM Wako Pure Chemical Corporation] was used instead of cobalt (II) acetate tetrahydrate, the addition amount of DMSO was changed from 7.27 mL to 7.60 mL, and the addition amount of a 25% methanol solution of TMAH was changed from 0.406 mL to 0.344 mL, then the proportion of the double salt in a dispersed state in the metal double salt dispersion liquid was determined through the same procedure as in Example 3. The results are shown in Table 1.

### (Production of metal oxide nanoparticle dispersion liquid)

The proportion of the oxide in a dispersed state in the metal oxide nanoparticle dispersion liquid was determined through the same procedure as in Example 3 except that the amount of pure water added at the time of producing the metal oxide nanoparticle dispersion liquid was changed to 0.055 mL, the heating in the oil bath was changed to 70°C for 1 hour, and the amount of methyl acetate added to the separated metal oxide nanoparticle dispersion liquid was changed to 7 times the volume of the separated liquid. The results are shown in Table 1.

### <Example 5>

### (Production of metal double salt dispersion liquid)

A metal double salt dispersion according to Example 5 was produced through the same procedure as in Example 3 except that 0.2 g of cerium (III) acetate monohydrate [manufactured by Nacalai tesque, Inc.] was used instead of cobalt (II) acetate tetrahydrate, the addition amount of DMSO was changed from 7.27 mL to 5.37 mL, and the addition amount of a 25% methanol solution of TMAH was changed from 0.406 mL to 0.377 mL, then the proportion of the double salt in a dispersed state in the metal double salt dispersion liquid was determined through the same procedure as in Example 3. The results are shown in Table 1.

### (Production of metal oxide nanoparticle dispersion liquid)

The proportion of the oxide in a dispersed state in the metal oxide nanoparticle dispersion liquid was determined through the same procedure as in Example 3 except that the amount of pure water added at the time of producing the metal oxide nanoparticle dispersion liquid was 0.082 mL, the heating in the oil bath was changed to 95°C for 1 hour, the amount of methyl acetate added to the separated metal oxide nanoparticle dispersion liquid was changed to 9 times the volume of the separated liquid, and the amount of methyl acetate added to the separated metal oxide nanoparticle dispersion liquid was changed to 9 times the volume of the separated liquid. The results are shown in Table 1.

### <Example 6>

### (Production of metal double salt dispersion liquid)

A metal double salt dispersion according to Example 6 was produced through the same procedure as in Example 3 except that 0.0971 g of manganese (II) acetate tetrahydrate [manufactured by FUJIFILM Wako Pure Chemical Corporation] was used in addition to 0.2 g of cobalt (II) acetate tetrahydrate, the addition amount of DMSO was changed from 7.27 mL to 5.48 mL, 5.48 mL of pyridine was further added in addition to DMSO as the organic solvent, and the addition amount of a 25% methanol solution of TMAH was changed from 0.406 mL to 0.6065 mL.

The proportion of the double salt in a dispersed state in the metal double salt dispersion liquid was determined through the same procedure as in Example 3 except that, as the solvent, only methyl acetate in an amount of 4 times the volume of the separated liquid was added to the separated metal double salt dispersion liquid and toluene was not added. The results are shown in Table 1.

### (Production of metal oxide nanoparticle dispersion liquid)

The proportion of the oxide in a dispersed state in the metal oxide nanoparticle dispersion liquid was determined through the same procedure as in Example 3 except that the amount of pure water added at the time of producing the metal oxide nanoparticle dispersion liquid was changed to 0.101 mL and the heating in the oil bath was changed to 95°C for 2 hours. The results are shown in Table 1.

### <Example 7>

### (Production of metal double salt dispersion liquid)

A metal double salt dispersion liquid according to Example 7 was produced through the same procedure as in Example 6 except that the amount of cobalt (II) acetate tetrahydrate was changed from 0.2 g to 1.0 g, the amount of manganese (II) acetate tetrahydrate was changed from 0.0971 g to 0.485 g, the addition amounts of DMSO and pyridine were each changed from 5.48 mL to 2.32 mL, and the addition amount of a 25% methanol solution of TMAH was changed from 0.6065 mL to 3.03 mL, then the proportion of the double salt in a dispersed state in the metal double salt dispersion liquid was determined. The results are shown in Table 1.

### (Production of metal oxide nanoparticle dispersion liquid)

The proportion of the oxide in a dispersed state in the metal oxide nanoparticle dispersion liquid was determined through the same procedure as in Example 6 except that the amount of pure water added at the time of producing the metal oxide nanoparticle dispersion liquid was 0 mL (that is, pure water was not added) and the heating in the oil bath was changed to 95°C for 7 hours. The results are shown in Table 1.

### <Example 8>

### (Production of metal double salt dispersion liquid)

A metal double salt dispersion liquid according to Example 8 was produced through the same procedure as in Example 7 except that the addition amounts of DMSO and pyridine were each changed from 2.32 mL to 3.30 mL, and 1.07 mL of diazabicycloundecene [manufactured by Tokyo Chemical Industry Co., Ltd.] was used instead of 3.03 mL of a 25% methanol solution of TMAH.

The proportion of the double salt in a dispersed state in the metal double salt dispersion liquid was determined through the same procedure as in Example 7 except that the solvent added to the separated metal double salt dispersion liquid was changed to methyl acetate in an amount of 4 times the volume of the separated liquid and toluene in an amount of 6 times the volume of the separated liquid. The results are shown in Table 1.

### (Production of metal oxide nanoparticle dispersion liquid)

The proportion of the oxide in a dispersed state in the metal oxide nanoparticle dispersion liquid was determined through the same procedure as in Example 7 except that the solvent added at the time of producing the metal oxide nanoparticle dispersion liquid was changed to methyl acetate in an amount of 4 times the volume of the separated liquid and toluene of 6 times the volume of the separated liquid. The results are shown in Table 1.

### <Example 9>

### (Production of metal double salt dispersion liquid)

A metal double salt dispersion liquid according to Example 9 was produced through the same procedure as in Example 3 except that the amount of cobalt (II) acetate tetrahydrate was changed from 0.2 g to 2.2 g, the addition amount of DMSO was changed from 7.27 mL to 14.45 mL, and 3.03 mL of a 25% methanol solution of TMAH was changed to 1.85 mL of diazabicycloundecene, then the proportion of the double salt in a dispersed state in the metal double salt dispersion liquid was determined through the same procedure as in Example 3. The results are shown in Table 1.

### (Production of metal oxide nanoparticle dispersion liquid)

The proportion of the oxide in a dispersed state in the metal oxide nanoparticle dispersion liquid was determined through the same procedure as in Example 3 except that the amount of pure water added at the time of producing the metal oxide nanoparticle dispersion liquid was changed to 0.023 mL, the heating in the oil bath was changed to 3 hours, air was introduced into the reaction solution while bubbling at a flow rate of 200 sccm at the time of the heating in the oil bath, and the solution added at the time of producing the separated metal oxide nanoparticle dispersion liquid was changed to methyl acetate in an amount of 4 times the volume of the separated liquid and toluene in an amount of 6 times the volume of the separated liquid. The results are shown in Table 1.

### <Example 10>

### (Production of metal double salt dispersion liquid)

A metal double salt dispersion liquid according to Example 10 was produced through the same procedure as in Example 9 except that the addition amount of DMSO was changed from 14.45 mL to 14.82 mL and 1.48 mL of diazabicyclononene [manufactured by Tokyo Chemical Industry Co., Ltd.] was used instead of 1.85 mL of diazabicycloundecene, then the proportion of the double salt in a dispersed state in the metal double salt dispersion liquid was determined through the same procedure as in Example 9. The results are shown in Table 1.

### (Production of metal oxide nanoparticle dispersion liquid)

The proportion of the oxide in a dispersed state in the metal oxide nanoparticle dispersion liquid was determined through the same procedure as in Example 9. The results are shown in Table 1.

### <Example 11>

### (Production of metal double salt dispersion liquid)

A metal double salt dispersion liquid according to Example 11 was produced through the same procedure as in Example 9 except that the addition amount of DMSO was changed from 14.45 mL to 14.74 mL and 1.55 mL of tetramethylguanidine [manufactured by Tokyo Chemical Industry Co., Ltd.] was used instead of 1.85 mL of diazabicycloundecene, then the proportion of the double salt in a dispersed state in the metal double salt dispersion liquid was determined through the same procedure as in Example 9. The results are shown in Table 1.

### (Production of metal oxide nanoparticle dispersion liquid)

The proportion of the oxide in a dispersed state in the metal oxide nanoparticle dispersion liquid was determined through the same procedure as in Example 9. The results are shown in Table 1.

### <Example 12>

### (Production of metal double salt dispersion liquid)

A metal double salt dispersion according to Example 12 was produced through the same procedure as in Example 9 except that 1.5 g of copper (II) acetate monohydrate was used in place of cobalt (II) acetate tetrahydrate, the addition amount of DMSO was changed from 14.45 mL to 12.61 mL, and the addition amount of diazabicycloundecene was changed from 1.85 mL to 1.35 mL, then the proportion of the double salt in a dispersed state in the metal double salt dispersion liquid was determined through the same procedure as in Example 9. The results are shown in Table 1.

### (Production of metal oxide nanoparticle dispersion liquid)

The proportion of the oxide in a dispersed state in the metal oxide nanoparticle dispersion liquid was determined through the same procedure as in Example 9 except that the amount of pure water added at the time of producing the metal oxide nanoparticle dispersion liquid was changed to 0.091 mL, the temperature of the oil bath was changed to 70°C, the heating in the oil bath was changed to 1 hour, and the introduction amount of the air into the reaction solution at the time of heating in the oil bath was changed to 0 sccm. The results are shown in Table 1.

### <Example 13>

### (Production of metal double salt dispersion liquid)

A metal double salt dispersion liquid according to Example 13 was produced through the same procedure as in Example 9 except that 1.5 g of manganese (II) acetate tetrahydrate was used in place of cobalt (II) acetate tetrahydrate, the addition amount of DMSO was changed from 14.45 mL to 10.38 mL, and the addition amount of diazabicycloundecene was changed from 1.85 mL to 0.932 mL, then the proportion of the double salt in a dispersed state in the metal double salt dispersion liquid was determined in the same procedure as in Example 9. The results are shown in Table 1.

### (Production of metal oxide nanoparticle dispersion liquid)

The proportion of the oxide in a dispersed state in the metal oxide nanoparticle dispersion liquid was determined through the same procedure as in Example 9 except that the amount of pure water added at the time of producing the metal oxide nanoparticle dispersion liquid was 0 mL, the temperature of the oil bath was changed to 70°C, and the heating in the oil bath was changed to 2 hours. The results are shown in Table 1.

### <Example 14>

### (Production of metal double salt dispersion liquid)

A metal double salt dispersion according to Example 14 was produced through the same procedure as in Example 9 except that 3.0 g of cerium (III) acetate monohydrate was used in place of cobalt (II) acetate tetrahydrate, 12.63 mL of NMP [super dehydration grade, manufactured by FUJIFILM Wako Pure Chemical Corporation] was used instead of DMSO, and the addition amount of diazabicycloundecene was changed from 1.85 mL to 3.34 mL, then the proportion of the double salt in a dispersed state in the metal double salt dispersion liquid was determined through the same procedure as in Example 9. The results are shown in Table 1.

### (Production of metal oxide nanoparticle dispersion liquid)

The proportion of the oxide in a dispersed state in the metal oxide nanoparticle dispersion liquid was determined through the same procedure as in Example 9 except that the amount of pure water added at the time of producing the metal oxide nanoparticle dispersion liquid was 0.045 mL. The results are shown in Table 1.

**[Table 1]**

| | Metal double salt dispersion liquid | | | | | | Metal oxide nanoparticle dispersion liquid | |
|---|---|---|---|---|---|---|---|---|
| | Metal carboxylate | Metal concentration [mol/L] | m | Strong base | Substance amount of strong base/substance amount of metal carboxylate | Proportion of double salt in dispersed state [mol%] | Product | Proportion of oxide in dispersed state [mol%] |
| Example 1 | Cu(CH₃COO)₂ | 0.1 | 2 | TMAH | 1. 00 | 89.3 | CuO | 71.7 |
| Example 2 | Cu(CH₃COO)₂ | 0.1 | 2 | TMAH | 1.75 | 12.6 | Cuo | 35.2 |
| Example 3 | Co (CH₃COO)₂ | 0.1 | 2 | TMAH | 1.20 | 37.4 | Co₃O₄ | 84.0 |
| Example 4 | Mn(CH₃COO)₂ | 0.1 | 2 | TMAH | 1. 00 | 97.0 | Mn₃O₄ | 70.0 |
| Example 5 | Ce(CH₃COO)₃ | 0.1 | 3 | TMAH | 1.50 | 84.8 | CeO₂ | 71.0 |
| Example 6 | Co (CH₃COO)₂ | 0.1 | 2 | TMAH | 1.20 | 41.9 | Co_{1.8}Mn_{1.2}O₄ | 68.6 |
| | Mn (CH₃COO)₂ | | | | | | | |
| Example 7 | Co (CH₃COO)₂ | 0.7 | 2 | TMAH | 1.20 | 35.0 | Co_{1.8}Mn_{1.2}O₄ | 22.0 |
| | Mn (CH₃COO)₂ | | | | | | | |
| Example 8 | Co (CH₃COO)₂ | 0.7 | 2 | DBU | 1.19 | 87.5 | Co_{1.8}Mn_{1.2}O₄ | 63.2 |
| | Mn (CH₃COO)₂ | | | | | | | |
| Example 9 | Co (CH₃COO)₂ | 0.5 | 2 | DBU | 1. 40 | 67.3 | Co₃O₄ | 59.2 |
| Example 10 | Co (CH₃COO)₂ | 0.5 | 2 | DBN | 1.36 | 64.8 | Co₃O₄ | 58.1 |
| Example 11 | Co (CH₃COO)₂ | 0.5 | 2 | TMG | 1. 40 | 61.5 | Co₃O₄ | 55.3 |
| Example 12 | Cu (CH₃COO)₂ | 0.5 | 2 | DBU | 1.20 | 72.5 | Cuo | 65.1 |
| Example 13 | Mn(CH₃COO)₂ | 0.5 | 2 | DBU | 1. 02 | 81.2 | Mn₃O₄ | 62.5 |
| Example 14 | Ce (CH₃COO)₃ | 0.5 | 3 | DBU | 2.50 | 77.3 | CeO₂ | 73.8 |
| Comparative Example 1 | Cu (CH₃COO)₂ | 0.1 | 2 | TMAH | 1. 90 | 0.0 | CuO | 1.3 |

### (Comparison of dispersion state between Example 1 and Comparative Example 1)

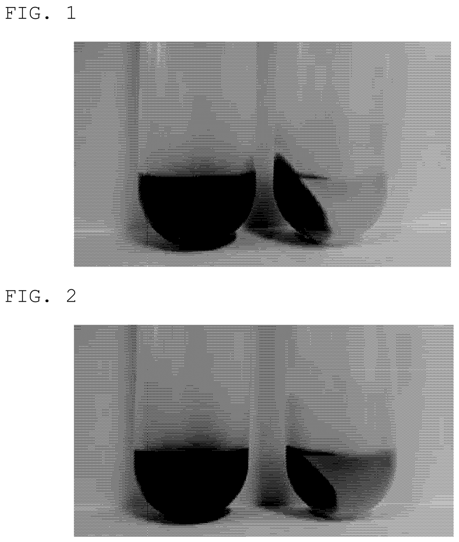

The dispersion states of the metal double salt dispersion liquid and the metal oxide nanoparticle dispersion liquid produced in Example 1 and Comparative Example 1 after being subjected to a centrifugal operation at a relative centrifugal force of 10,000 G for 5 minutes were checked. The results are shown in FIGS. 1 and 2. FIG. 1 is a photograph of the metal double salt dispersion liquids according to Example 1 and Comparative Example 1 after being subjected to a centrifugal operation, and FIG. 2 is a photograph of the metal oxide nanoparticle dispersion liquids according to Example 1 and Comparative Example 1 after being subjected to a centrifugal operation.

FIG. 1 shows that precipitation was observed in the metal double salt dispersion liquid according to Comparative Example 1 (right side), whereas no precipitation was observed in the metal double salt dispersion liquid according to Example 1 (left side). FIG. 2 shows that precipitation was observed in the metal oxide nanoparticle dispersion liquid according to Comparative Example 1 (right side), whereas no precipitation was observed in the metal oxide nanoparticle dispersion liquid according to Example 1 (left side).

From these results, it was confirmed that the dispersibility of the metal double salt in the metal double salt dispersion liquid according to Example 1 and the dispersibility of the metal oxide nanoparticles in the metal oxide nanoparticle dispersion liquid were high. It is considered that the metal oxide nanoparticles are colloidally dispersed and stabilized in the metal oxide nanoparticle dispersion liquid that is produced using the metal double salt dispersion of the present invention.

### <Example 15>

### (Checking of metal double salt)

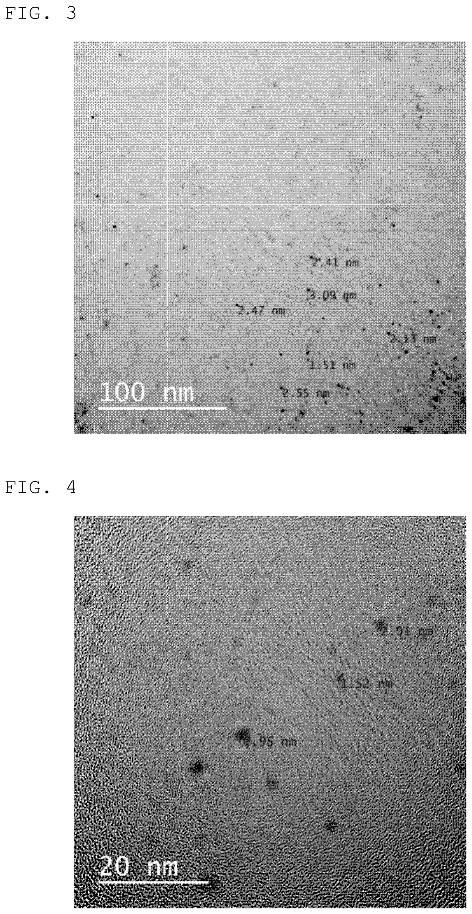

Ethanol in an amount of 3.49 mL was added to 0.4 g of copper (II) acetate monohydrate, and they were mixed at room temperature. To this solution, 0.299 mL of diazabicycloundecene was added dropwise and mixed at room temperature to produce a metal double salt dispersion liquid. Subsequently, the dispersion liquid was diluted with ethanol so that the metal concentration was 0.001 M. One drop of the diluted liquid was added onto a slide film that is a support film for TEM observation and dried, then the place where the dispersion liquid was dropped on the slide film was observed by TEM. The results are shown in FIGS. 3 and 4. FIGS. 3 and 4 are TEM images of the metal double salt according to Example 15.

From FIG. 3, it was confirmed that the metal double salt was present as a particulate matter having a particle size of about 1.5 nm to about 3.1 nm in a dry state in the metal double salt dispersion liquid. The average particle size of 30 metal double salts randomly selected in the field of view was 2.1 nm. From FIG. 4, grating fringes were observed in the particulate matter, and it was confirmed that the metal double salt was a crystalline substance.

### (Checking of average particle size of metal double salt using DLS and SAXS)

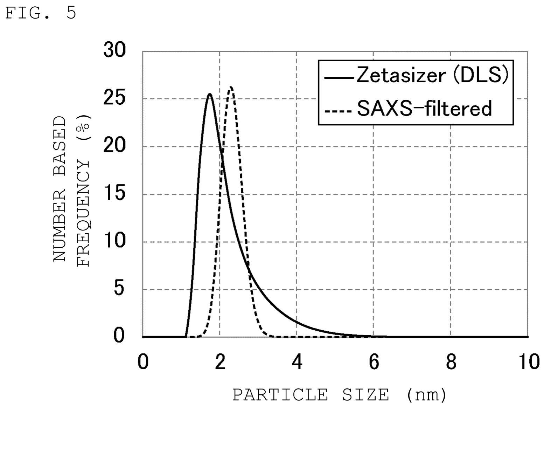

For reference, the particle size of the particles contained in the metal double salt dispersion liquid prepared in the checking of the metal double salt was measured by a dynamic light scattering method (DLS) and an X-ray small angle scattering method (SAXS). The results are shown in FIG. 5. FIG. 5 is a spectrum showing a particle size distribution of particles contained in the metal double salt dispersion liquid according to Example 15.

The left side (solid line) is the measurement result of DLS, and the right side (broken line) is the measurement result of SAXS. From FIG. 5, it was confirmed that the results of DLS and SAXS roughly correspond to the average particle size of the metal double salt measured by TEM.

### (Example 16)

### (Production of metal double salt dispersion liquid)

Cobalt (II) acetate tetrahydrate and manganese (II) acetate tetrahydrate were weighed to be 6 : 4 in molar ratio of metal components, added to DMSO [super dehydration grade, manufactured by FUJIFILM Wako Pure Chemical Corporation], and mixed at room temperature, whereby the metal salt was mixed with the organic solvent. To this mixture, 1.2 times the amount of DBU in terms of molar ratio with respect to the total substance amount of the metal component was added to obtain a metal double salt dispersion liquid.

### (Production of metal oxide nanoparticle dispersion liquid)

Pure water in an amount 0.5 times as much as the total substance amount of the metal components in terms of molar ratio was added to the obtained metal double salt dispersion liquid, and the mixture was heated to 95°C and held for 2.5 hours while air was bubbled in the air atmosphere, whereby a metal oxide nanoparticle dispersion liquid was obtained.

### (Purification of metal oxide nanoparticle dispersion liquid)

Methyl acetate was added to the obtained metal oxide nanoparticle dispersion liquid, and the mixture was centrifuged at 10,000 G for 5 minutes to precipitate metal oxide nanoparticles. The supernatant solution was removed, and 2-propanol and diethylene glycol monoethyl ether were added to the precipitate to disperse the precipitate, then heptane was added, and the mixture was centrifuged at a centrifugal force of 10,000 G for 5 minutes to precipitate metal oxide nanoparticles. The supernatant solution was removed, and 2-propanol and diethylene glycol monoethyl ether were added to the precipitate to disperse the precipitate, then heptane was added, and the mixture was centrifuged at a centrifugal force of 10,000 G for 5 minutes to precipitate metal oxide nanoparticles. After the supernatant solution was removed, ethylene glycol monopropyl ether was added to the precipitate to disperse the precipitate, whereby a purified metal oxide nanoparticle dispersion liquid was obtained.

### (Example 17)

A metal oxide nanoparticle dispersion liquid according to Example 17 was produced through the same procedure as in Example 16 except that DBN of the same substance amount as DBU was used instead of DBU.

### (Example 18)

A metal oxide nanoparticle dispersion liquid according to Example 18 was produced through the same procedure as in Example 16 except that TMG of the same substance amount as DBU was used instead of DBU.

### (Example 19)

A metal oxide nanoparticle dispersion liquid according to Example 19 was produced through the same procedure as in Example 16 except that manganese (II) acetate tetrahydrate was not used and the addition amount of DBU was changed to 1.4 times the amount of cobalt in terms of molar ratio.

### (Example 20)

A metal oxide nanoparticle dispersion liquid according to Example 20 was produced through the same procedure as in Example 19 except that DBN of the same substance amount as DBU was used instead of DBU.

### (Example 21)

A metal oxide nanoparticle dispersion liquid according to Example 21 was produced through the same procedure as in Example 19 except that TMG of the same substance amount as DBU was used instead of DBU.

### (Example 22)

A metal oxide nanoparticle dispersion liquid according to Example 22 was produced through the same procedure as in Example 16 except that cobalt (II) acetate tetrahydrate was not used, the addition amount of DBU was changed to 1.0 times the amount of manganese in terms of molar ratio, pure water was not added, and the heating temperature was changed to 70°C.

### (Example 23)

A metal oxide nanoparticle dispersion liquid according to Example 23 was produced through the same procedure as in Example 22 except that DBN of the same substance amount as DBU was used instead of DBU.

### (Example 24)

A metal oxide nanoparticle dispersion liquid according to Example 24 was produced through the same procedure as in Example 22 except that TMG of the same substance amount as DBU was used instead of DBU.

### (Example 25)

A metal oxide nanoparticle dispersion liquid according to Example 25 was produced through the same procedure as in Example 16 except that copper (II) acetate monohydrate was used instead of manganese (II) acetate tetrahydrate and cobalt (II) acetate tetrahydrate, the addition amount of pure water was changed to 2.0 times the amount of copper in terms of molar ratio, the heating temperature was changed to 75°C, and air bubbling during stirring was not performed.

### (Example 26)

A metal oxide nanoparticle dispersion according to Example 26 was produced through the same procedure as in Example 25 except that DBN of the same substance amount as DBU was used instead of DBU.

### (Example 27)

A metal oxide nanoparticle dispersion liquid according to Example 27 was produced through the same procedure as in Example 25 except that TMG of the same substance amount as DBU was used instead of DBU.

### (Example 28)

A metal oxide nanoparticle dispersion liquid according to Example 28 was produced through the same procedure as in Example 16 except that iron (II) acetate anhydride was used instead of manganese (II) acetate tetrahydrate and cobalt (II) acetate tetrahydrate, and the addition amount of pure water was changed to 4.0 times the amount of iron in terms of molar ratio.

### (Example 29)

A metal oxide nanoparticle dispersion liquid according to Example 29 was produced through the same procedure as in Example 28 except that DBN of the same substance amount as DBU was used instead of DBU.

### (Example 30)

A metal oxide nanoparticle dispersion liquid according to Example 30 was produced through the same procedure as in Example 28 except that TMG of the same substance amount as DBU was used instead of DBU.

### (Example 31)

A metal oxide nanoparticle dispersion liquid according to Example 31 was produced through the same procedure as in Example 16 except that indium acetate (III) anhydride was used instead of manganese (II) acetate tetrahydrate and cobalt (II) acetate tetrahydrate, the addition amount of DBU was changed to 2.0 times the amount of indium in terms of molar ratio, the addition amount of pure water was changed to 4.0 times the amount of indium in terms of molar ratio, and air bubbling was not performed during stirring.

### (Example 32)

A metal oxide nanoparticle dispersion liquid of Example 32 was produced through the same procedure as in Example 16 except that cerium (III) acetate monohydrate was used instead of manganese (II) acetate tetrahydrate and cobalt (II) acetate tetrahydrate, the addition amount of DBU was changed to 2.5 times the molar ratio with respect to cerium, and the addition amount of pure water was changed to 1.0 times the molar ratio with respect to indium.

### (Example 33)

A metal oxide nanoparticle dispersion liquid according to Example 33 was produced through the same procedure as in Example 32 except that DBN of the same substance amount as DBU was used instead of DBU.

### (Example 34)

A metal oxide nanoparticle dispersion liquid according to Example 34 was produced through the same procedure as in Example 32 except that TMG of the same substance amount as DBU was used instead of DBU.

### (Comparative Example 2)

A metal oxide nanoparticle dispersion liquid according to Comparative Example 2 was produced through the same procedure as in Example 16 except that a methanol solution (concentration 2.0 M) of lithium hydroxide monohydrate in the same substance amount as DBU was used instead of DBU.

### (Measurement of base amount)

For the metal oxide nanoparticle dispersion liquid according to Examples 16 to 34 and Comparative Example 2, the molar ratio of the content of the organic base having an amidine skeleton or a guanidine skeleton contained in the purified metal oxide nanoparticle dispersion liquid to the substance amount of all metal components contained in the metal oxide nanoparticle dispersion liquid was measured by gas chromatography. The results are shown in Table 2.

### (Measurement of temporal stability of dispersibility of metal oxide nanoparticle dispersion liquid)

The purified metal oxide nanoparticle dispersion liquid according to Examples 16 to 34 and Comparative Example 2 was subjected to a centrifugal operation at a relative centrifugal force of 10,000 G for 5 minutes, then the supernatant was separated, and the concentration of the metal component contained in the supernatant was measured. Thereafter, the metal oxide nanoparticle dispersion liquid was sealed in a glass bottle and allowed to stand for one week under conditions of a relative humidity of 50% and a temperature of 25°C, thereafter the concentration of the metal component was measured through the same procedure, and the reduction width of the change rate of the metal oxide nanoparticles in a dispersed state in the metal oxide nanoparticle dispersion liquid before and after standing was calculated. The results are shown in Table 2.

The temporal stability was evaluated as "good" when the reduction width in the change rate of the metal oxide nanoparticles in a dispersed state before and after standing for one week was less than 1.0%, and the temporal stability was evaluated as "poor" when the reduction width was 1.0% or more.

### (Measurement of crystal phase of metal oxide nanoparticles)

The metal oxide nanoparticle dispersion liquid according to Examples 16 to 34 and Comparative Example 2 was dried to obtain a powder, the crystal phase of the metal oxide nanoparticles was identified using a powder X-ray diffraction method, and the composition of the metal oxide nanoparticles was determined from the crystal phase. The results are shown in Table 2.

### (Measurement of average particle size of metal oxide nanoparticles)

The average particle size (D50) of the metal oxide nanoparticles dispersed in the metal oxide nanoparticle dispersion liquid according to Examples 16 to 34 and Comparative Example 2 was measured using a dynamic light scattering method. The results are shown in Table 2.

**[Table 2]**

| | Metal oxide nanoparticle | | Organic base having amidine skeleton or guanidine skeleton | | Reduction width of change rate of metal oxide nanoparticle in dispersed state [%] | Temporal stability of dispersibility of metal oxide nanoparticle |
|---|---|---|---|---|---|---|
| | Composition | Average particle size [nm] | Type | Molar ratio to substance amount of all metal component | | |
| Example 16 | Co_{2.8}Mn_{1.2}O₄ | 3.9 | DBU | 0.0035 | 0.1 | Good |
| Example 17 | Co_{2.8}Mn_{1.2}O₄ | 3.8 | DBN | 0.0043 | 0.2 | Good |
| Example 18 | Co_{2.8}Mn_{1.2}O₄ | 4.0 | TMG | 0.0071 | 0.2 | Good |
| Example 19 | Co₃O₄ | 5.1 | DBU | 0.0051 | 0.1 | Good |
| Example 20 | Co₃O₄ | 5.0 | DBN | 0.0062 | 0.2 | Good |
| Example 21 | Co₃O₄ | 5.2 | TMG | 0.0075 | 0.3 | Good |
| Example 22 | Mn₃O₄ | 5.7 | DBU | 0.0037 | 0.3 | Good |
| Example 23 | Mn₃O₄ | 5.5 | DBN | 0.0050 | 0.5 | Good |
| Example 24 | Mn₃O₄ | 5.8 | TMG | 0.0055 | 0.5 | Good |
| Example 25 | Cuo | 5.6 | DBU | 0.022 | 0.1 | Good |
| Example 26 | Cuo | 5.9 | DBN | 0.021 | 0.2 | Good |
| Example 27 | Cuo | 5.5 | TMG | 0.025 | 0.2 | Good |
| Example 28 | Fe₂O₃ | 3.0 | DBU | 0.0056 | 0.1 | Good |
| Example 29 | Fe₂O₃ | 2.8 | DBN | 0.0058 | 0.3 | Good |
| Example 30 | Fe₂O₃ | 2.9 | TMG | 0.0063 | 0.2 | Good |
| Example 31 | In₂O₃ | 7.3 | DBU | 0.0032 | 0.5 | Good |
| Example 32 | CeO₂ | 2.3 | DBU | 0.0066 | 0.2 | Good |
| Example 33 | CeO₂ | 2.4 | DBN | 0.0075 | 0.3 | Good |
| Example 34 | CeO₂ | 2.5 | TMG | 0.0079 | 0.3 | Good |
| Comparative Example 2 | Co_{2.8}Mn_{1.2}O₄ | 3.9 | - | 0 | 15 | Poor |

From the results in Table 2, it was found that the metal oxide nanoparticle dispersion liquids according to Examples 16 to 34 using an organic base having an amidine skeleton or a guanidine skeleton as an organic base are excellent in temporal stability of the dispersibility of metal oxide nanoparticles.

## Claims

1. A metal oxide nanoparticle dispersion liquid comprising:
metal oxide nanoparticles; and
an organic base having an amidine skeleton or a guanidine skeleton, wherein a molar ratio of a content of the organic base to a content of a metal component constituting the metal oxide nanoparticles is 0.03 or less.

2. The metal oxide nanoparticle dispersion liquid according to claim 1, wherein the organic base includes at least one compound selected from the group consisting of diazabicycloundecene, diazabicyclononene, and tetramethylguanidine.

3. The metal oxide nanoparticle dispersion liquid according to any one of claims 1 to 2, wherein the metal component constituting the metal oxide nanoparticles includes at least one metal selected from the group consisting of Cu, Mn, Co, Ce, Fe, and In.

4. The metal oxide nanoparticle dispersion liquid according to any one of claims 1 to 3, wherein the metal component constituting the metal oxide nanoparticles includes Ce.

5. A method for producing a metal oxide nanoparticle dispersion liquid according to any one of claims 1 to 4, the method comprising:
a preparation step of preparing a metal double salt dispersion liquid by adding a strong base to a metal salt dispersion liquid including a metal carboxylate and an organic solvent; and
a heating step of heating the metal double salt dispersion liquid in a presence of water to obtain a metal oxide nanoparticle dispersion liquid,
wherein
in the preparation step, when a metal element constituting the metal carboxylate has a valence m, the strong base has a substance amount with respect to a substance amount of the metal carboxylate of 0.4m or more and 0.9m or less, and
the strong base includes an organic base having an amidine skeleton or a guanidine skeleton.

6. The method for producing a metal oxide nanoparticle dispersion liquid according to claim 5, wherein the organic base includes at least one compound selected from the group consisting of diazabicycloundecene, diazabicyclononene, and tetramethylguanidine.

7. The method for producing a metal oxide nanoparticle dispersion liquid according to claim 5 or 6, wherein a metal component constituting the metal oxide nanoparticles includes at least one metal selected from the group consisting of Cu, Mn, Co, Ce, Fe, and In.
